# EUROPÄISCHE PATENTSCHRIFT

(11) **EP 2 381 907 B1**
(45) Veröffentlichungstag und Bekanntmachung des Hinweises auf die Patenterteilung: **30.10.2013**
(21) Anmeldenummer: 10703617.0
(22) Anmeldetag: 29.01.2010
(51) Int. Cl.: A61F 13/06

(54) **SPRUNGGELENKBANDAGE**
ANKLE JOINT BANDAGE
BANDAGE POUR L'ARTICULATION DE LA CHEVILLE

(30) Priorität: 29.01.2009 DE 102009006628
(43) Veröffentlichungstag der Anmeldung: 02.11.2011
(73) Patentinhaber: Bauerfeind AG, 07937 Zeulenroda-Triebes (DE)
(72) Erfinder: BÄTZ, Ronny, 07937 Vogtländisches Oberland (DE); STIER, Gerald, 07937 Langenwetzendorf (DE); RÖBELT, Gerhard, 07950 Zeulenroda-Triebes (DE)
(74) Vertreter: Bardehle, Heinz
(86) Internationale Anmeldenummer: PCT/EP2010/000556
(87) Internationale Veröffentlichungsnummer: WO 2010/086181

(56) Entgegenhaltungen:
- US-A- 4 085 746
- US-A- 5 139 479

## Beschreibung

Die Erfindung bezieht sich auf eine Sprunggelenkbandage mit einem sich mindestens über Mittelfuß und Sprunggelenk erstreckenden Strumpf und einem den Fuß umfassenden Befestigungsband.

Eine derartige Bandage ist in der DE 4318 791 A1 beschrieben. Diese bekannte Sprunggelenkbandage weist einen sich über Mittelfuß und Sprunggelenk erstreckenden elastischen Strumpf auf, der zur Halterung von Stützorganen dient. Dabei handelt es sich um einen steigbügelartig verlaufenden Zügel aus reiß- und zugfestem Material, der beidseitig im Wesentlichen über das Sprunggelenk und den Fersenbereich des betreffenden Fußabschnittes umfasst. Darüber hinaus ist ein elastischer Gurt vorgesehen, der medial vom Fersenbereich her über diesen sohlenseitig verläuft und danach den Fußrücken überquert, um schließlich oberhalb des Sprunggelenkes den Unterschenkel umfasst und auf dem Fußrücken endet, wo er an dem den Fußrücken schräg überquerenden Band festgelegt ist. Diese Gestaltung der bekannten Sprunggelenkbandage ist auf eine Einwirkung auf den Fersenbereich ausgerichtet, wodurch eine besondere Beeinflussung im Bereich des Mittelfußes, insbesondere dessen Stabilisierung, weitgehend außer Betracht bleibt.

Aus der DE 200 05 742 U1 ist eine weitere Sprunggelenkbandage bekannt, bei der ein einziges teilweise elastisches, teilweise unelastisches Band von der Lateralseite einer Fußkante über den Mittelfußrücken gelegt ist und danach oberhalb des Knöchels den Unterschenkel umfasst. Es existiert damit nur eine einfache Überquerung des Fußes, von der somit nur unsymmetrisch gegen ein Umknicken des Fußes in dieser einen Richtung stabilisiert werden kann. Eine umfassende Stabilisierung des Fußes ist mit dieser Sprunggelenkbandage also nicht möglich.

Eine Bandage ist in der US 4085746 beschrieben.

Der Erfindung liegt die Aufgabe zugrunde, eine Sprunggelenkbandage zu schaffen, mit der vor allem ein schräg nach innen gerichtetes und gleichzeitig drehendes Abknicken des Fußes aufgefangen werden kann, womit einerseits diesem gefährlichen Bewegungsmechanismus entgegengewirkt und andererseits auch durch eine entsprechende nervliche Signalisierung insbesondere dem Sportler das Gefühl gegeben werden soll, seinen Fuß in der betreffenden Richtung nicht besonders zu belasten. Erfindungsgemäß geschieht dies dadurch, dass das Befestigungsband medial im Mittelfußbereich als elastischer Gurt an einem Ausgangspunkt am Strumpf angebracht ist und von dort der Gurt fußsohlenseitig um den Mittelfuß umlegbar und den Mittelfußrücken kreuzend von der medialen Seite her den Unterschenkel oberhalb des Knöchels umfasst, danach von lateral kommend wieder den Mittelfußrücken überquert und von der medialen Seite her nochmals den Mittelfuß fußsohlenseitig umläuft und an einem Bandagenbereich oberhalb der Fußsohle befestigbar ist.

Es ist möglich, dass der Gurt nach nochmaligem fußsohlenseitigen Umlaufen des Mittelfußes zum Bereich des Anschlusspunktes mit einem Klettverschluss geführt ist. Man kann den Gurt auch so führen, dass er nach nochmaligem fußsohlenseitigen Umlaufen des Mittelfußes zur medialen Seite des den Unterschenkel oberhalb des Knöchels umfassenden Gurtbereich geführt und dort mit einem Klettverschluss an dem Gurt befestigt ist.

Aufgrund der Umschlingung des Mittelfußes unter Einbeziehung des Unterschenkels oberhalb des Knöchels liegt im Prinzip eine nach Art einer Acht verlaufende Bandführung vor, die insbesondere Fußabbiegungen schräg zur eigentlichen normalen Fußrichtung auffängt. Damit ergibt sich eine besonders stabile Halterung für den Fuß unter Ermöglichung einer gewissen Beweglichkeit, soweit die Elastizität des Gurtes diese zulässt, womit erreicht wird, dass der Träger der Bandage bei entsprechender Belastung seines Fußes frühzeitig auf Fehlbewegungen aufmerksam gemacht wird. Diese Gestaltung der Sprunggelenkbandage und insbesondere die Führung des Befestigungsbandes wird hinsichtlich seiner Wirkung dadurch noch unterstützt, dass das Befestigungsband nach Umlaufen des Unterschenkels nochmals von der medialen Seite her den Mittelfuß umläuft und an der Oberseite des Mittelfußes befestigbar ist. Durch das nochmalige Umlaufen des Mittelfußes wird die Wirkung des Befestigungsbandes verstärkt und somit die Gefahr einer Verletzung des Fußes bei entsprechenden Fehlbewegungen verringert. Darüber hinaus ergibt sich aufgrund der doppelten Führung des Befestigungsbandes nach Art einer Acht ein wesentlich vergrößerter Tragekomfort.

Vorteilhaft gestaltet man das Befestigungsband so, dass es aus einem unelastischen und einem elastischen Teil besteht, wobei der elastische Teil der medialen Befestigungsstelle und der unelastische Teil dem Klettverschluss zugewandt ist. Aufgrund dieser Gestaltung lässt sich das Befestigungsband unter Ausnutzung des elastischen Teils satt unter einer gewissen Spannung am Fuß anlegen, woraufhin dann der zusätzlich angelegte unelastische Teil dafür sorgt, dass dem Fuß über diese elastische Halterung des Fußes mit dem unelastischen Teil des Befestigungsbandes keine weitere wesentliche Bewegungsmöglichkeit gegeben wird. Im Wesentlichen handelt es sich dabei auch um eine Bequemlichkeit des Anlegens der Bandage, wobei zunächst das Umlegen um den Mittelfuß mit dem elastischen Teil beginnt und nach Anlegen des elastischen Teils unter dem Gefühl einer nicht unangenehmen Anspannung dann erst das Anlegen des unelastischen Teiles folgt.

In den Figuren sind Ausführungsbeispiele der Erfindung dargestellt. Es zeigen:
- Fig. 1: die Sprunggelenkbandage mit zweifachem Umlaufen des Mittelfußes und Befestigung des Gurtendes am Ausgangspunkt des Gurtes,
- Fig. 2: die Sprunggelenkbandage ähnlich derjenigen gemäß Fig. 1, allerdings mit Befestigung des Gurtendes im Bereich des Unterschenkels oberhalb des Knöchels.

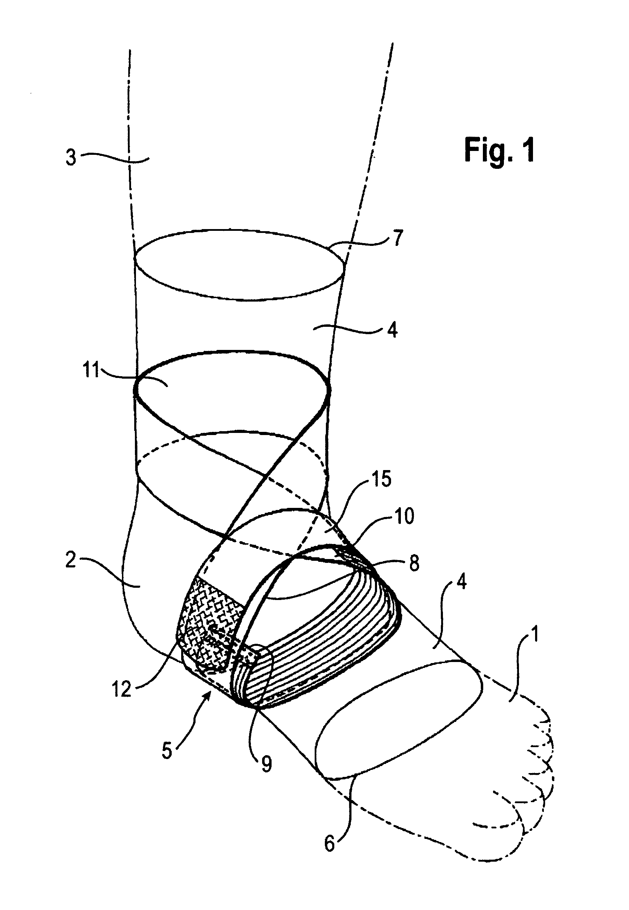

In der Figur 1 ist ein linker Fuß 1 dargestellt, der über die Ferse 2 in den Unterschenkel 3 übergeht. Über den Fuß 1 ist der Strumpf 4 gezogen, der sich über den Bereich des Mittelfußes 5 und die Ferse 2 auf den Unterschenkel 3 erstreckt. Das fußseitige Ende des Strumpfes 4 ist mit 6 und das unterschenkelseitige Ende mit 7 bezeichnet.

Die Sprunggelenkbandage weist das aus dem Gurt 8 bestehende Befestigungsband auf, das mit seinem einen Ende medial im Bereich des Mittelfußes 5 oberhalb der Fußsohle mit dem Strumpf 4 an der Befestigungsstelle 9 verbunden ist. Von dieser Befestigungsstelle 9 umläuft der Gurt 8 die Fußsohle und kreuzt danach von lateral kommend den Mittelfuß 5 im Bereich 10, umschlingt dann mit der Schlinge 11 den Unterschenkel 3 und damit auch den Strumpf 4. Nach der Umschlingung verläuft dann der Gurt 8 wieder über den Mittelfußrücken über die Befestigungsstelle 9 hinweg, umschlingt nochmals die Fußsohle, um danach den Mittelfußrücken im Bereich des Mittelfußes 5 zu überqueren, bis der Gurt 8 schließlich zur Befestigungsstelle 9 reicht und hier mit seinem Ende mittels eines durch die Kreuzschraffierung angedeuteten Klettverschlusses 12 am Gurt 8 befestigt ist.

Es ergibt sich damit eine doppelte Umschlingung des Mittelfußes durch den Gurt 8 und außerdem,eine Umschlingung des Unterschenkels 3 im Bereich oberhalb des Knöchels in Art einer Acht, wodurch dem Fuß und insbesondere seinem Gelenk im Knöchel eine besonders hohe Stabilität gegeben ist.

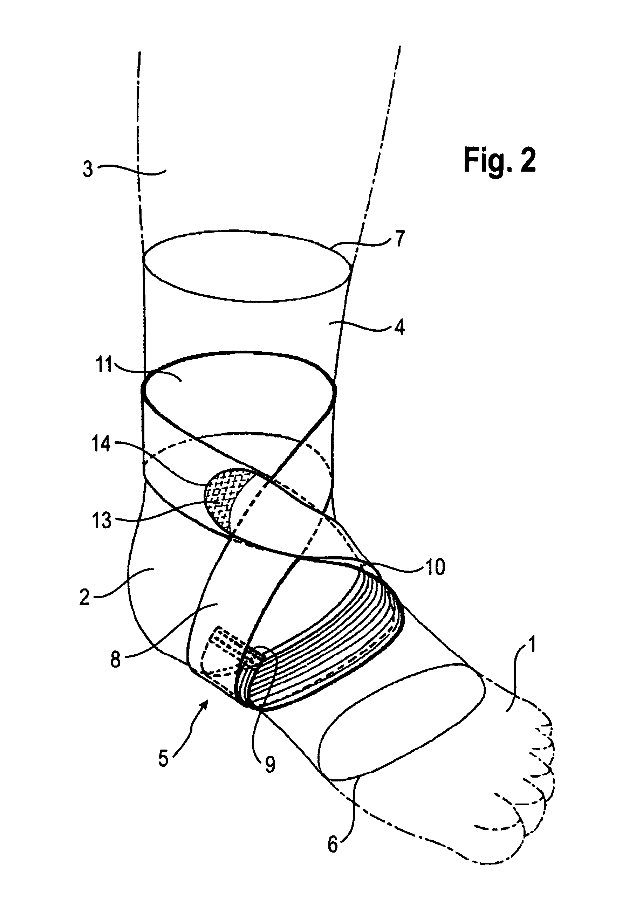

In der Figur 2 ist eine Abwandlung der Gestaltung der Sprunggelenkbandage gemäß Figur 1 dargestellt, bei der der Ausgangspunkt des Gurtes 8 genauso gewählt ist, wie dies in der Figur 1 dargestellt ist, jedoch das Gurtende nach zweimaliger Umschlingung des Mittelfußes 5 in Richtung auf die Schlinge 11 des Gurtes 8 gerichtet und mit einem Klettverschluss 13 an dem Gurt 8 in dessen Bereich der Umfassung des Unterschenkels an der Stelle 14 befestigt ist.

Diese letztere Befestigungsgestalt des Gurtes 8 kann gegebenenfalls zu einer einfacheren Methode des Anlegens der Sprunggelenkbandage führen. Außerdem vermeidet man mit dieser Befestigung eine Zusammenfassung von Anfang und Ende des Gurtes, die zu einer unerwünschten Dickstelle führen kann. Therapeutisch besteht zwischen den beiden Sprunggelenkbandagen gemäß Figur 1 und Figur 2 kein grundlegender Unterschied.

Wie bereits oben erwähnt, kann man den Gurt aus einem unelastischem und einem elastischen Teil aufbauen. Dies ist in den Figuren 1 und 2 dadurch dargestellt, dass der unmittelbar an die Befestigungsstelle 9 anschließende Teil des Gurtes 8 mit parallelen, längs verlaufenden Linien versehen ist, durch die eine Elastizität dargestellt werden soll. Wie ersichtlich, endet diese Zeichnungsgestaltung nach der erstmaligen Umschlingung der Fußsohlenseite des Mittelfußes 5, wonach dann vom Gurtbereich 10 an die Längsschraffierung weggelassen ist, wodurch die Unelastizität des Gurtes 8 in diesem Bereich dargestellt werden soll.

## Patentansprüche

1. Sprunggelenkbandage mit einem sich mindestens über Mittelfuß und Sprunggelenk erstreckenden Strumpf (4) und einem den Fuß (1) umfassenden Befestigungsband, **dadurch gekennzeichnet, dass** das Befestigungsband medial im Mittelfußbereich als elastischer Gurt (8) an einem Ausgangspunkt (9) am Strumpf (4) angebracht ist und von dort der Gurt (8) fußsohlenseitig um den Mittelfuß umlegbar und den Mittelfußrücken kreuzend von der medialen Seite her den Unterschenkel (3) oberhalb des Knöchels umfasst, danach von lateral kommend wieder den Mittelfußrücken überquert und von der medialen Seite her nochmals den Mittelfuß fußsohlenseitig umläuft und an einem Bandagenbereich oberhalb der Fußsohle befestigbar ist.

2. Sprunggelenkbandage nach Anspruch 1, **dadurch gekennzeichnet, dass** der Gurt (8) nach nochmaligem fußseitigen Umlaufen des Mittelfußes zum Bereich des Ausgangspunktes (9) mit einem Klettverschluss (12) geführt ist.

3. Sprunggelenkbandage nach Anspruch 1, **dadurch gekennzeichnet, dass** der Gurt (8) nach nochmaligem fußsohlenseitigen Umlaufen des Mittelfußes zur medialen Seite des den Unterschenkel (3) oberhalb des Knöchels umfassenden Gurtbereich geführt und dort mit einem Klettverschluss (13) an dem Gurt (8) befestigt ist.

4. Sprunggelenkbandage nach einem der Ansprüche 1 bis 3, **dadurch gekennzeichnet, dass** der Gurt aus einem unelastischen und einem elastischen Teil besteht, wobei der elastische Teil dem medialen Ausgangspunkt (9) und der unelastische Teil dem Klettverschluss (12; 13) zugewandt ist.

## Claims

1. An ankle joint bandage including a stocking (4) which extends at least across the middle foot and the ankle joint, and a fixing strap surrounding the foot (1) **characterized in that** the fixing strap, in the form of an elastic strap (8), is medially connected to an anchoring point (9) on the stocking (4) in the middle foot region, from where the strap (8) may then be passed around the middle foot on the sole side and, after crossing the middle of the instep, then loops around the lower leg (3) above the ankle from the medial side, then laterally crosses the middle of the instep again and runs around the middle foot on the sole side once more and may be fixed to a bandage region above the sole.

2. The ankle joint bandage of claim 1 **characterized in that** the strap (8), after running around the middle foot on the sole side again, is passed to the region of the starting point (9) by means of a hook-and-loop fastener (12).

3. The ankle joint bandage of claim 1 **characterized in that** the strap (8), after running around the middle foot on the sole side again, is passed to the medial side of the strap portion which loops around the lower leg (3) above the ankle and is fixed to the strap (8) there by means of a hook-and-loop fastener (12).

4. The ankle joint bandage of one of claims 1 to 3 **characterized in that** the strap consists of a non-elastic and an elastic portion, with the elastic portion facing the medial starting point (9) and the non-elastic portion facing the hook-and-loop fastener (12; 13).

## Revendications

1. Bandage d'articulation de la cheville avec une chaussette (4) qui s'étend au moins par-dessus le métatarse et l'articulation de la cheville et un ruban de fixation qui entoure le pied (1), **caractérisé en ce que** le ruban de fixation est attaché latéralement en tant que sangle élastique (8) dans la région du métatarse à un point de départ (9) sur la chaussette (4) et, à partir de là, la sangle (8) peut être rabattue dans le sens de la plante du pied autour du métatarse et entoure le dos du métatarse en croisé à partir du côté médian ainsi que le bas de la jambe (3) au-dessus de la cheville, puis, latéralement, passe de nouveau par-dessus le dos du métatarse et entoure de nouveau, à partir du côté médian, le métatarse du côté de la plante du pied pour être rattaché à une zone du bandage au-dessus de la plante du pied.

2. Bandage d'articulation de la cheville selon la revendication 1, **caractérisé en ce que** la sangle (8), après avoir entouré normalement le métatarse du côté du pied, dispose d'une fermeture velcro (12) dans la zone du point de départ (9).

3. Bandage d'articulation de la cheville selon la revendication 1, **caractérisée en ce que** la sangle (8), après avoir entouré normalement le métatarse du côté de la plante du pied, est dirigé vers le côté médian de la zone de la sangle entourant le bas de la jambe (3) au-dessus de la cheville, puis y est fixé à l'aide d'une fermeture velcro (13) située sur la sangle (8).

4. Bandage d'articulation de la cheville selon l'une des revendications 1 à 3, **caractérisé en ce que** la sangle est composée d'une partie élastique et d'une partie non élastique, la partie élastique étant orientée vers le point de départ médian (9) et la partie non élastique étant orientée vers la fermeture velcro (12; 13).
